# EUROPÄISCHE PATENTSCHRIFT

(11) **EP 0 713 485 B1**
(45) Veröffentlichungstag und Bekanntmachung des Hinweises auf die Patenterteilung: **15.04.1998**
(21) Anmeldenummer: 94926134.1
(22) Anmeldetag: 29.07.1994
(51) Int. Cl.: C07D 231/20, A01N 43/56, C07C 69/716, C07C 69/738

(54) **THIOCARBAMOYLVERBINDUNGEN ALS MIKROBIZIDE**
THIOCARBAMOYL COMPOUNDS AS MICROBICIDES
COMPOSES DU THIOCARBAMOYLE MICROBICIDES

(30) Priorität: 11.08.1993 DE 4326904; 31.03.1994 DE 4411243
(43) Veröffentlichungstag der Anmeldung: 29.05.1996
(73) Patentinhaber: BAYER AG, 51368 Leverkusen (DE)
(72) Erfinder: HEUER, Lutz, D-47800 Krefeld (DE); WACHTLER, Peter, D-51061 Köln (DE); KUGLER, Martin, D-42799 Leichlingen (DE); SCHRAGE, Heinrich, D-47800 Krefeld (DE)
(86) Internationale Anmeldenummer: EP9402534
(87) Internationale Veröffentlichungsnummer: WO9504722

(56) Entgegenhaltungen:
- EP-A- 0 515 934
- JOURNAL OF THE AMERICAN CHEMICAL SOCIETY., Bd.104, Nr.13, 30. Juni 1982, GASTON, PA US Seiten 3727 - 3729 R. TAMURA ET AL. 'Palladium(0)-Catalyzed Allylic Alkylation and Amination of Allylnitroalkanes'
- JOURNAL OF THE CHEMICAL SOCIETY, CHEMICAL COMMUNICATIONS, Nr.21, 6. November 1974, LETCHWORTH GB Seiten 908 - 909 K. YAMADA ET AL. 'Total Synthesis of the Alkaloid (+/-)-Geissoschizine'
- BULLETIN DE LA SOCIETE CHIMIQUE DE FRANCE, Nr.10, 1969, PARIS FR Seiten 3694 - 3698 R. JACQUIER ET AL. 'Synthèse et équilibre céto-énolique de beta-cétoesters'
- JOURNAL OF ORGANIC CHEMISTRY., Bd.21, Nr.9, 16. Oktober 1956, EASTON US Seiten 1022 - 1024 N.P. BUU-HOI ET AL. 'Benzocoumarins of Steroid-like Structure'
- TETRAHEDRON LETTERS., Bd.34, Nr.52, 24. Dezember 1993, OXFORD GB Seiten 8509 - 8512 T. OHSHIMA ET AL. 'Manganese(III)-Based Oxidative Free-Radical Reaction of alpha-Allyl-beta-Keto Ester with Molecular Oxygen'
- Jerry March "Advanced Organic Chemistry",3 Auflage,S.438

## Beschreibung

Die Anmeldung betrifft neue Thiocarbamoyl-Verbindungen, deren Herstellung und deren Verwendung zum Schutz von technischen Materialien.

Thiocarbamoyl-Verbindungen und deren Verwendung im Materialschutz sind bekannt und werden z.B. in (DE 4 117 385) beschrieben. Diese Verbindungen haben aber den Nachteil, daß sie eine nicht immer ausreichende mikrobizide Wirkung haben und darüberhinaus aus den zu schützenden Materialien ausgewaschen werden. Ein Langzeitschutz ist durch diese bekannten Verbindungen daher nicht gewährleistet.

Gegenstand der Anmeldung sind daher die neuen Thiocarbamoyl-Verbindungen der Formel (I) 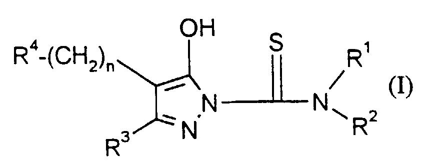 in denen
- R¹, R², R³: jeweils unabhängig voneinander für Wasserstoff oder Methyl stehen und
- R⁴: für Cyclopentyl oder Cyclopentenyl steht,
- n: für die Zahl 0 oder 1 steht,
sowie deren Metallsalzkomplexe und Säureadditionsprodukte.

Besonders bevorzugt sind Verbindungen der Formel (I), in denen R¹, R² und R³ für Wasserstoff stehen und n für die Zahl 0 steht und insbesondere die Verbindung der Formel (I), in der R¹, R², R³ für Wasserstoff stehen, n für die Zahl 0 steht, R⁴ für Cyclopentyl steht.

Die Verbindungen der Formel (I) werden erhalten, indem man Formylsäurederivate der Formel (II) 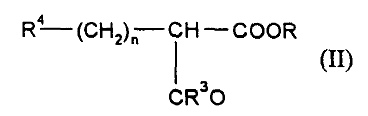 in denen n, R³ und R⁴ die oben angegebenen Bedeutungen haben und R für Methyl, Ethyl, n-, i-Propyl oder n-, i-, s- oder t-Butyl steht mit Thiosemicarbaziden der Formel (III)

NH₂-NH-CS-NR¹R² (III)

gegebenenfalls in Gegenwart eines Verdünnungs- bzw. Lösungsmittels und/oder einer Base umsetzt.

Pro Mol α-Formylessigsäurederivat setzt man dabei vorzugsweise 0,8 bis 1,0 Mol Thiosemicarbazid zu.

Als Basen werden vorzugsweise Natriumhydroxid, Kaliumhydroxid oder Kalium-tert.-butylat, vorzugsweise in etwa äquimolaren Mengen zugesetzt.

Als Lösungsmittel bzw. Verdünnungsmittel kommen vor allem Alkohole wie Ethanol oder aromatische Kohlenwasserstoffe wie Toluol in Frage.

Die Kondensationsreaktion wird innerhalb eines größeren Temperaturbereichs durchgeführt. Für die zuerst ablaufende Thiosemicarbazonbildung wird bei Temperaturen von 20 bis 110°C, vorzugsweise 60 bis 90°C, gearbeitet. Die nach der Basenzugabe ablaufende Cyclokondensationsreaktion wird bei Temperaturen von 20 bis 100°C, vorzugsweise 20 bis 40°C, vorgenommen.

Die erfindungsgemäßen 1-Thiocarbamoyl-Verbindungen der Formel (I) werden nach bekannten Methoden aus den Reaktionsgemischen isoliert. Man geht im allgemeinen so vor, daß man die Reaktionsgemische vom Lösungsmittel befreit und den Rückstand mit wäßriger Salzsäure behandelt. Die dabei ausfallenden Pyrazole werden durch Absaugen abgetrennt. Es ist jedoch auch möglich, das Reaktionsgemisch unmittelbar in einen großen Überschuß verdünnter Salzsäure einzugießen und die als Niederschlag sich abscheidenden Pyrazole abzufiltrieren.

Die Thiosemicarbazide der Formel (III) sind bekannt oder nach allgemein bekannten Herstellungsverfahren erhältlich.

Die Formylsäurederivate der Formel (II) werden erhalten, in dem man Ester der Formel (IV)

R⁴-(CH₂)ₙ-CH₂-COOR (IV)

in denen R⁴, n und R die oben angegebene Bedeutung haben,
mit Basen, wie z.B. NaH, in Lösungs- bzw. Verdünnungsmitteln, wie z.B.Cyclohexan und DMF, mit Aminsäureestern, wie Methyl- oder Ethylester formyliert.

Die Formylsäurederivate der Formel (II) werden auch erhalten, indem man entsprechende α,β-ungesättigte Ester nach allgemein bekannten Methoden, wie z.B. in EP-417.597 oder DE-2.643.205 beschrieben, hydroformyliert.

Die Wirkstoffe der Formel (I) und die erfindungsgemäßen Mittel weisen eine starke mikrobizide Wirkung auf und können zur Bekämpfung von unerwünschte Mikroorganismen praktisch eingesetzt werden. Die Wirkstoffe der Formel (I) und die erfindungsgemäßen Mittel sind zum Schutz von technischen Materialien gegen Befall und Zerstörung durch unerwünschte Mikroorganismen geeignet.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Anstrichmittel.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe der Formel (I) können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Bevorzugt werden die erfindungsgemäßen Wirkstoffe der Formel (I) zum Schutz von Anstrichen gegen Befall und Zerstörung durch unerwünschte Mikroorganismen eingesetzt.

Unter Anstrich ist im vorliegenden Zusammenhang eine aus Anstrichstoffen hergestellte Beschichtung auf einem Untergrund zu verstehen. Der Anstrich kann mehr oder weniger in den Untergrund eingedrungen sein. Er kann aus einer oder mehreren Schichten bestehen und durch Verfahren wie Streichen, Spritzen, Tauchen, Fluten oder ähnliche Verfahren hergestellt werden.

Die Verbindungen der Formeln (I) werden in die Anstrichmittel oder in Vorprodukte zur Herstellung der Anstrichmittel nach üblichen Methoden, z.B. durch Vermischen der Wirkstoffe mit den anderen Komponenten, eingearbeitet.

Erfindungsgemäße Anstrichmittel enthalten daher neben mindestens einem fungiziden Wirkstoff der Formel (I) allgemein übliche Anstrichkomponenten in z.B. flüssiger, pastöser oder pulverförmiger Form wie z.B.
- Farbmittel, wie Pigmente oder Farbstoffe, bevorzugt Pigmente. Beispielsweise genannt sei Titandioxid, Zinkoxid und Eisenoxid.
- Bindemittel, wie beispielsweise oxidativ trocknende Alkydharze, Vinylpolymerisate und Vinylcopolymerisate, Acrylpolymerisate und Acrylcopolymerisate, Kunststoffpulver, Novolacke, Aminoharze, Polyesterharze, Epoxidharze, Silikonharze, Isocyanatharze bevorzugt sind Vinylpolymerisate und Vinylcopolymerisate, Acrylpolymerisate und Acrylcopolymerisate und andere in Wasser verdünnbaren Anstrichstoffen verwendbare Bindemittel.

Daneben enthalten die Anstriche gegebenenfalls folgende Zusatzstoffe
- Füllstoffe, wie beispielsweise Schwerspat, Calcit, Dolomit und Talk,
- Lösemittel, wie beispielsweise Alkohole, Ketone, Ester, Glykolether und aliphatische sowie aromatische Kohlenwasserstoffe,
- sowie Verdickungs- und Thixotropiermittel, Dispergier-und Netzmittel, Trockenstoffe, Hautverhütungsmittel, Verlaufmittel, Antischaummittel, Korrosionsinhibitoren, UV-Absorber, Duftstoffe, Antistatika, Frostschutzmittel.

Als Anstrichmittel bzw. Vorprodukte zur Herstellung von Anstrichmitteln seien vorzugsweise folgende genannt:
- Leime und Klebstoffe auf Basis der bekannten tierischen, pflanzlichen oder synthetischen Rohstoffe.
- Kunststoffdispersionen wie Latexdispersionen oder Dispersionen auf Basis anderer Polymere.
- Stärkelösungen, -dispersionen oder -slurries oder andere auf Basis von Stärke hergestellte Produkte wie z.B. Druckverdicker.
- Slurries anderer Rohstoffe wie Farbpigmente (z.B. Eisenoxidpigmente, Rußpigmente, Titandioxidpigmente) oder Slurries von Füllstoffen wie Kaolin oder Calciumcarbonat.
- Betonadditive beispielsweise auf Basis von Melasse oder Ligninsulfonaten.
- Bitumenemulsionen.
- Vor- und Zwischenprodukte der chemischen Industrie, z.B. bei der Farbstoffproduktion und -lagerung.
- Tinten oder Tuschen.
- Dispersionsfarben für die Anstrichindustrie.
- Schichten und Appreturen.

Die Wirksamkeit und das Wirkungsspektrum der Wirkstoffe der Formeln (I) bzw. die daraus herstellbaren Mittel, Vorprodukte oder ganz allgemein Formulierungen kann erhöht werden, wenn gegebenenfalls weitere antimikrobiell wirksame Verbindungen. Fungizide, Bakterizide, Herbizide, Insektizide oder andere Wirkstoffe zur Vergrößerung des Wirkungsspektrums oder Erzielung besonderer Effekte wie z.B. des zusätzlichen Schutzes vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:

### Triazole wie:

Amitrole, Azocyclotin, BAS 480F, Bitertanol, Difenoconazole, Fenbuconazole, Fenchlorazole, Fenethanil, Fluquinconazole, Flusilazole, Flutriafol, Imibenconazole, Isozofos, Myclobutanil, Metconazole, Epoxyconazole, Paclobutrazol, Penconazole, Propioconazole, (±)-cis-1-(4-chlorphenyl)-2-(1H-1,2,4-triazol-1-yl)-cycloheptanol, Tetraconazole, Triadimefon, Triadimenol, Triapenthenol, Triflumizole, Triticonazole, Uniconazole sowie deren Metallsalze und Säureaddukte.

### Imidazole wie:

Imazalil, Pefurazoate, Prochloraz, Triflumizole, 2-(1-tert-Butyl)-1-(2-chlorphenyl)-3-(1,2,4-triazol-1-yl)-propan-2-ol, Thiazolcarboxanilide wie 2',6'-Dibromo-2-methyl-4-trifluoromethoxy-4'-trifluoromethyl-1,3-thiazole-5-carboxanilide, 1-Imidazolyl-1-(4'-chlorophenoxy)-3,3-dimethylbutan-2-on sowie deren Metallsalze und Säureaddukte.

Methyl(E)-2-[2-[6-(2-cyanophenoxy)pyrimidin-4-yloxy]phenyl]3-methoxyacrylate, methyl(E)-2-[2-[6-(2-thioamidophenoxy)pyrimidin-4-yloxy]phenyl]-3 -methoxyacrylate, methyl(E)-2-[2-[6-(2-fluorophenoxy)pyrimidin-4-yloxy]phenyl]-3-methoxyacrylate, methyl(E)-2-[2-[6-(2,6-difluorophenoxy)pyrimidin-4-yloxy]phenyl]-3-methoxyacrylate, methyl(E)-2-[2-[3-(pyrimidin-2-yloxy)phenoxy]phenyl]-3-methoxyacrylate, methyl(E)-2-[2-[3 -(5-methylpyrimidin-2-yloxy)-phenoxy]phenyl]-3 -methoxyacrylatemethyl(E)-2-[2-[3-(phenyl-sulfonyloxy)phenoxy]phenyl]-3-methoxyacrylate,methyl(E)-2-[2-[3-(4-nitrophenoxy)phenoxy]phenyl]-3-methoxyacrylate, methyl(E)-2-[2-phenoxyphenyl]-3-methoxyacrylate, methyl(E)-2-[2-(3,5-dimethylbenzoyl)pyrrol-1-yl]-3-methoxyacrylate, methyl(E)-2-[2-(3-methoxyphenoxy)phenyl]-.3-methoxyacrylate, methyl(E)-2-[2-(2-phenylethen-1-yl)-phenyl]-3-methoxyacrylatenethyl(E)-2-[2-(3,5-dichl orophenoxy)pyridin-3-yl]-3-methoxyacrylate, methyl(E)-2-(2-(3-(1,1,2,2-tetrafluoroethoxy)phenoxy)phenyl)-3-methoxyacrylate, methyl(E)-2-(2-[3-(alpha-hydroxybenzyl)phenoxy]phenyl)-3-methoxyacrylate, methyl(E)-2-(2-(4-phenoxypyridin-2-yloxy)phenyl)-3-methoxyacrylatemethyl(E)-2-[2-(3-n-propyloxyphenoxy)phenyl]3-methoxyacrylate, methyl(E)-2-[2-(3-isopropyloxyphenoxy)phenyl]-3-methoxyacrylate, methyl(E)-2-[2-[3-(2-fluorophenoxy)pehnoxy]phenyl]-3-methoxyacrylate, methyl(E)-2-[2-(3-ethoxyphenoxy)phenyl]-3-methoxyacrylatemethyl(E)-2-[2-(4-tert.-butylpyridin-2-yloxy)phenyl]-3-methoxyacrylate, methyl(E)-2-[2-[3-(3-cyanophenoxy)phenoxy]phenyl]-3-methoxyacrylatepethyl(E)-2-[2-(3-methylpyridin-2-yloxymethyl)phenyl]-3-methoxyacrylatepethyl(E)-2-[2-[6-(2-methylphenoxy)pyrimidin-4-yloxy]phenyl]-3-methoxyacrylate,methyl(E)-2-[2-(5-bromopyridin-2-yloxymethyl)phenyl]-3-methoxyacrylate, methyl(E)-2-[2-(3-(3-iodopyridin-2-yloxy)phenoxy)phenyl]-3-methoxyacrylate, methyl(E)-2-[2-[6-(2-chloropyridin-3-yloxy)pyrimidin-4-yloxy]phenyl]-3-methoxyacrylate, (E),(E)methyl-2-[2-(5,6-dimethylpyrazin-2-ylmethyloximinomethyl)phenyl]-3-methoxyacrylate(E)-methyl-2-{2-[6-(6-methylpyridin-2-yloxy)pyrimidin-4-yloxy]phenyl}-3-methoxyacrylate, (E),(E)methyl-2-{2-(3-methoxyphenyl)methyloximinomethyl]phenyl}-3-methoxyacrylate, (E)methyl-2-{2-(6-(2-azidophenoxy)-pyrimidin-4-yloxy]phenyl}3-methoxyacrylate,(E),(E)methyl-2-{2-[6-phenylpyrimidin-4-yl)-methyloximinomethyl]phenyl}-3-methoxyacrylate, (E),(E)methyl-2-{2-[(4-chlorophenyl)-methyloximinomethyl]phenyl}-3-methoxyacrylate, (E)methyl-2-{2-[6-(2-n-propylphenoxy)-1,3,5-triazin-4-yloxy]phenyl}-3-methoxyacrylate, (E),(E)methyl-2 {2-[(3-nitrophenyl)methyloximinomethyl]phenyl}-3-methoxyacrylate;

### Succinat-Dehydrogenase Inhibitoren wie:

Fenfuram, Furcarbanil, Cyclafluramid, Furmecyclox, Seedvax, Metsulfovax, Pyrocarbolid, Oxycarboxin, Shirlan, Mebenil (Mepronil), Benodanil, Flutolanil (Moncut);
Naphthalin-Derivate wie Terbinafine, Naftifine, Butenafine, 3-Chloro-7-(2-aza-2,7,7-trimethyl-oct-3-en-5-in);
Sulfenamide wie Dichlofluanid, Tolylfluanid, Folpet, Fluorfolpet; Captan, Captofol;
Benzimidazole wie Carbendazim, Benomyl, Furathiocarb, Fuberidazole, Thiophonatmethyl, Thiabendazole oder deren Salze;
Morpholinderivate wie Tridemorph, Fenpropimorph, Falimorph, Dimethomorph, Dodemorph, Aldimorph, Fenpropidin und ihre arylsulfonsauren Salze, wie z.B. p-Toluolsulfonsäure und p-Dodecylphenyl-sulfonsäure;
Dithiocarbamate, Cufraneb, Ferbam, Mancopper, Mancozeb, Maneb, Metam, Metiram, Thiram Zeneb, Ziram;
Benzthiazole wie 2-Mercaptobenzothiazol;
Benzamide wie 2,6-Dichloro-N-(4-trifluoromethylbenzyl)-benzamide;
Borverbindungen wie Borsäure, Borsäureester, Borax;
Formaldehyd und Formaldehydabspaltende Verbindungen wie Benzylalkoholmono(poly)-hemiformal, Oxazolidine, Hexa-hydro-S-triazine, N-Methylolchloracetamid, Paraformadehyd, Nitropyrin, Oxolinsäure, Tecloftalam;
Tris-N-(cyclohexyldiazeniumdioxy)-aluminium, N-(Cyclo-hexyldiazeniumdioxy)-tributylzinn bzw. K-Salze, Bis-N-(cydohexyldiazeniumdioxy)-kupfer;
N-Methylisothiazolin-3 -on,S-Chlor-N-methylisothiazolin-3 -on,4,5-Dichloro-N-octylisothiazolin-3-on, N-Octyl-isothiazolin-3-on, 4,5-Trimethylen-isothiazolinone, 4,5-Benzisothiazolinone, N-Methylolchloracetamid;
Aldehyde wie Zimtaldehyd, Formaldehyd, Glutardialdehyd, β-Bromzimtaldehyd;
Thiocyanate wie Thiocyanatomethylthiobenzothiazol, Methylenbisthiocyanat, usw;
quartäre Ammoniumverbindungen wie Benzyldimethyltetradecylmnmoniumchlorid, Benzyldimethyldodecylammoniumchlorid, Didecyldimethaylammoniumchlorid;
Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-hexylcarbamat, 3-Iod-2-propinyl-cyclohexylcarbamat, 3-Iod-2-propinyl-phenylcarbamat;
Phenolderivate wie Tribromphenol, Tetrachlorphenol, 3-Methyl-4-chlorphenol, 3,5-Dimethyl-4-chlorphenol, Phenoxyethanol, Dichlorphen, o-Phenylphenol, m-Phenylphenol, p-Phenylphenol, 2-Benzyl-4-chlorphenol und deren Alkali- und Erdalkalimetallsalze;
Mikrobizide mit aktivierter Halogengruppe wie Chloracetamid, Bronopol, Bronidox, Tectamer wie 2-Brom-2-nitro-1,3-propandiol, 2-Brom-4'-hydroxy-acetophenon, 2,2-Dibrom-3-nitril-propionamid, 1,2-Dibrom-2,4-dicyanobutan, β-Brom-β-nitrostyrol;
Pyridine wie 1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Metallseifen wie Zinn-, Kupfer-, Zinknaphtenat, -octoat, 2-ethylhexanoat, -oleat, -phosphat, -benzoat;
Metallsalze wie Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
Oxide wie Tributylzinnoxid, Cu₂O, CuO, ZnO;
Dialkyldithiocarbamate wie Na- und Zn-Salze von Dialkyldithiocarbamaten, Tetramethylthiuramdisulfid, Kalium-N-methyl-dithiocarbamat;
Nitrilewie2,4,5,6-Tetrachlorisophthalodinitril,Dinatrium-cyano-dithioimidocarbamat; Chinoline wie 8-Hydroxychinolin und deren Cu-Salze;
Mucochlorsäure, 5-Hydroxy-2(5H)-furanon;
4,5-Dichlorodithiazolinon, 4,5-Benzdithiazolinon, 4,5-Trimethylendithiazolinon, 4,5-Dichlor-(3H)-1,2-dithiol-3-on, 3,5-Dimethyl-tetrahydro-1,3,5-thiadiazin-2-thion, N-(2-p-Chlorbenzoylethyl)-hexaminiumchloridKalium-N-hydroxymethyl-N'-methyldithiocarbamat,
2-Oxo-2-(4-hydroxy-phenyl)acethydroximsäure-chlorid,
Phenyl-(2-chlor-cyan-vinyl)sulfon,
Phenyl-(1,2-dichlor-2-cyan-vinyl)sulfon;
Ag, Zn oder Cu-haltige Zeolithe allein oder eingeschlossen in polymere Wirkstoffe.

Ganz besonders bevorzugt sind Mischungen mit
Azaconazole, Bromuconazole, Cyproconazole, Dichlobutrazol, Diniconazole, Hexaconazole, Metaconazole, Penconazole, Propiconazole, Tebuconazole, Methyl-(E)-methoximino[α-(o-tolyloxy)-o-tolyl)]acetate, Methyl-(E)-2-{2-[6-(2-cyanphenoxy)-pyrimidin-4-yl-oxy]phenyl}-3-methoxyacrylat, Methfuroxam, Carboxin, Fenpiclonil, 4-(2,2-Difluoro-1,3-benzodioxol-4-yl)- 1H-pyrrol-3-carbonitril, Butenafine, Imazalil, N-Methyl-isothiazolin-3-on, 5-Chlor-N-methylisothiazolin-3-on, N-Octylisothiazolin-3-on, Benzisothiazolinone, N-(2-Hydroxypropyl)-amino-methanol, Benzylalkohol(hemi)-formal, Glutaraldehyd, Omadine, Dimethyldicarbonat.

Desweiteren werden auch gut wirksame Mischungen mit den folgenden Wirkstoffen hergestellt:

### Fungizide:

Acypetacs, 2-Aminobutane, Ampropylfos, Anilazine, Benalaxyl, Bupirimate, Chinomethionat, Chloroneb, Chlozolinate, Cymoxanil, Dazomet, Diclomezine, Dichloram, Diethofencarb, Dimethirimol, Diocab, Dithianon, Dodine, Drazoxolon, Edifenphos, Ethirimol, Etridiazole, Fenarimol, Fenitropan, Fentin acetate, Fentin Hydroxide, Ferimzone, Fluazinam, Fluromide, Flusulfamide, Flutriafol, Fosetyl, Fthalide, Furalaxyl, Guazatine, Hymexazol, Iprobenfos, Iprodione, Isoprothiolane, Metalaxyl, Methasulfocarb, Nitrothal-isopropyl, Nuarimol, Ofurace, Oxadiyl, Perflurazoate, Pencycuron, Phosdiphen, Pimaricin, Piperalin, Procymidone, Propamocarb, Propineb, Pyrazophos, Pyrifenox, Pyroquilon, Quintozene, Tar Oils, Tecnazene, Thicyofen, Thiophanate-methyl, Tolclofos-methyl, Triazoxide, Trichlamide, Tricyclazole, Triforine, Vinclozolin.

### Insektizide:

Phosphorsäureester wie Azinphos-ethyl, Azinphos-methyl, α-1(4-Chlorphenyl)-4-(O-ethyl, S-propyl)phosphoryloxy-pyrazol, Chlorpyrifos, Coumaphos, Demeton, Demeton-S-methyl, Diazinon, Dichlorvos, Dimethoate, Ethoate, Ethoprophos, Etrimfos, Fenitrothion, Fenthion, Heptenophas, Parathion, Parathion-methyl, Phosalone, Phoxim, Pirimiphos-ethyl, Pirimiphos-methyl, Profenofos, Prothiofos, Sulfprofos, Triazophos und Trichlorphon;
Carbamate wie Aldicarb, Bendiocarb, α-2-(1-Methylpropyl)-phenylmethylcarbamat, Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb;
OrganosiliciumverbindungenvorzugsweiseDimethyl(phenyl)silyl-methyl-3-phenoxybenzylether wie Dimethyl-(4-ethoxyphenyl)-silylmethyl-3-phenoxybenzylether oder (Dimethylphenyl)-silyl-methyl-2-phenoxy-6-pyridylmethyletherwiez.B.Dimethyl-(9-ethoxy-phenyl)-silylmethyl-2-phenoxy-6-pyridylmethylether oder [(Phenyl)-3-(3-phenoxyphenyl)-propyl](dimethyl)-silane wie z.B. (4-Ethoxyphenyl)-[3-(4-fluoro-3-phenoxyphenyl-propyl]dimethyl-silan, Silafluofen;
Pyrethroide wie Allethrin, Alphamethrin, Bioresmethrin, Byfenthrin, Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluor-methylvinyl)cyclopropancarboxylat, Fenpropathrin, Fenfluthrin, Fenvalerate, Flucythrinate, Flumethrin, Fluvalinate, Permethrin, Resmethrin und Tralomethrin;
Nitroimine und Nitromethylene wie 1-[(6-Chlor-3-pyridinyl)-methyl]-4,5-dihydro-N-nitro-1H-imidazol-2-amin (Imidacloprid), N-[(6-Chlor-3-pyridyl)methyl-]N²-cyano-N¹-methylacetamide (NI-25);
Abamectin, AC 303, 630, Acephate, Acrinathrin, Alanycarb, Aldoxycarb, Aldrin, Amitraz, Azamethiphos, Bacillus thuringiensis, Phosmet, Phosphamidon, Phosphine, Prallethrin, Propaphos, Propetamphos, Prothoate, Pyraclofos, Pyrethrins, Pyridaben, Pyridafenthion, Pyriproxyfen, Quinalphos, RH-7988, Rotenone, Sodium fluoride, Sodium hexafluorosilicate, Sulfotep, Sulfuryl fluoride, Tar Oils, Teflubenzuron, Tefluthrin, Temephos, Terbufos, Tetrachlorvinphos, Tetramethrin, O-2-tert.-Butylpyrimidin-5-yl-o-isopropyl-phosphorothiate, Thiocyclam, Thiofanox, Thiometon, Tralomethrin, Triflumuron, Trimethacarb, Vamidothion, Verticillium Lacanii, XMC, Xylylcarb, Benfuracarb, Bensultap, Bifenthrin, Bioallethrin, MERbioallethrin (S)-cyclopentenyl isomer, Bromophos, Bromophos-ethyl, Buprofezin, Cadusafos, Calcium Polysulfide, Carbophenothion, Cartap, Chinomethionat, Chlordane, Chlorfenvinphos, Chlorfluazuron, Chlormephos, Chloropicrin, Chlorpyrifos, Cyanophos, Beta-Cyfluthrin, Alpha-cypermethrin, Cyophenothrin, Cyromazine, Dazomet, DDT, Demeton-S-methylsulphon, Diafenthiuron, Dialifos, Dicrotophos, Diflubenzuron, Dinoseb, Deoxabenzofos, Diaxacarb, Disulfoton, DNOC, Empenthrin, Endosulfan, EPN, Esfenvalerate, Ethiofencarb, Ethion, Etofenprox, Fenobucarb, Fenoxycarb, Fensulfothion, Fipronil, Flucycioxuron, Flufenprox, Flufenoxuron, Fonofos, Formetanate, Formothion, Fosmethilan, Furathiocarb, Heptachlor, Hexaflumuron, Hydramethylnon, Hydrogen Cyanide, Hydroprene, IPSP, Isazofos, Isofenphos, Isoprothiolane, Isoxathion, Iodfenphos, Kadethrin, Lindane, Malathion, Mecarbam, Mephosfolan, Mercurous, chloride, Metam, Metarthizium, anisopliae, Methacrifos, Methamidophos, Methidathion, Methiocarb, Methoprene, Methoxychlor, Methyl isothiocyanate, Metholcarb, Mevinphos, Monocrotophos, Naled, Neodiprion sertifer NPV, Nicotine, Omethoate, Oxydemeton-methyl, Pentachlorophenol, Petroleum oils, Phenothrin, Phenthoate, Phorate;

### Molluscicide:

Fentinacetate, Metaldehyde, Methiocarb. Niclosamide, Thiodicarb, Trimethacarb.

### Algicide:

Coppersulfate, Dichlororphen, Endothal, Fentinacetate, Quinoclamine.

### Herbicide:

acetochlor, acifluorfen, aclonifen, acrolein, alachlor, alloxydim, ametryn, amidosulfuron, amitrole, ammonium sulfamate, anilofos, asulam atrazine, aziptrotryne, benazolin, benfluralin, benfuresate, bensulfuron, bensulfide, bentazone, benzofencap, benzthiazuron, bifenox, bilanafos, borax, dichlorprop, dichlorprop-P, diclofop, diethatyl, difenoxuron, difenzoquat, diflufenican, dimefuron, dimepiperate, dimethachlor, dimethametryn, dimethipin, dimethylarsinic acid, dinitramine, dinoseb, dinoseb, dinoseb acetate, dinoseb, bromacil, bromobutide, bromofenoxim, bromoxynil, butachlor, butamifos, fuenachlor, butralin, butylate, carbetamide, CGA 184927, chlormethoxyfen, chloramben, chlorbromuron, chlorbutam, chlorfurenol, chloridazon, chlorimuron, chlornitrofen, chloroacetic acid, achloropicrin, chlorotoluron, chloroxuron, chlorprepham, chlorsulfuron, chlorthal, chlorthiamid, cinmethylin, cinofulsuron, clethodim, clomazone, clomeprop, clopyralid, cyanamide, cyanazine, dinoseb acetate, dinoterb, diphenamid, dipropetryn, diquat, dithiopyr, diduron, DNOC, PPX-A 788, DPX-E96361, DSMA, eglinazine, endothal, EPTC, esprocarb, ethalfluralin, ethidimuron, ethofumesate, fenoxaprop, fenoxaprop-P, fenuron, flamprop, flamprop-M, flazasulfuron, fluazifop, fluazifop-P, fluchloralin, flumeturon, fluorocgycofen, fluoronitrofen, flupropanate, flurenol, fluridone, flurochloridone, fluoroxypyr, cycloate, cycloxydim, 2,4-D, daimuron, dalapon, dazomet, 2,4-DB, desmedipham, desmetryn, dicamba, dichlorbenil, isoproturon, isouron, isoxaben, isoxapyrifop, lactofen, lenacil, linuron, LS830556, MCPA, MCPA-thioethyl, MCPB, mecoprop, mecoprop-P, mefenacet, mefluidide, metam, metamitron, metazachlor, methabenzthiazuron, methazole, methoproptryne, methyldymron, methylisothiocyanate, metobromuron, fomosafen, fosamine, furyloxyfen, glufosinate, glyphosate, haloxyfop, hexazinone, imazamethabenz, imazapyr, imazaquin, imazethapyr, ioxynil, isopropalin, propyzamide, prosulfocab, pyrazolynate, pyrazolsulfuron, pyrazoxyfen, pyributicarb, pyridate, quinclorac, quinmerac, quinocloamine, quizalofop, quzizalofop-P, S-23121, sethoxydim, sifuron, simazine, simetryn, SMY 1500, sodium chlorate, sulfometuron, tar oils, TCA, metolachlor, metoxuron, metribzin, metsulfuron, molinate, monalide, monolinuron, MSMA, naproanilide, napropamide, naptalam, neburon, nicosulfuron, nipyraclofen, norflurazon, orbencarb, oaryzalin, oxadiazon, oxyfluorfen, paraquat, pebulate, pendimethalin, pentachlorophenol, pentaochlor, petroleum oils, phenmedipham, picloram, piperophos, pretilachlor, primisulfuron, prodiamine, proglinazine, propmeton, prometryn, propachlor, tebutam, tebuthiuron, terbacil, terbumeton, terbuthylazine, terbutryn, thiazafluoron, thifensulfuron, thiobencarb, thiocarbazil, tioclorim, tralkoxydim, tri-allate, triasulfuron, tribenzuron, triclopyr, tridiphane, trietazine, trifluralin, IBI-C4874 vernolate, propanil, propaquizafop, propazine, propham.

Die Gewichtsverhältnisse der Wirkstoffe in diesen Wirkstoffkombinationen können in relativ großen Bereichen variiert werden.

Vorzugsweise erhalten die Wirkstoffkombinationen den Wirkstoff der Formel (I) zu 0,1 bis 99,9 %, insbesondere zu 1 bis 75 %, besonders bevorzugt 5 bis 50 %, wobei der Rest zu 100 % durch einen oder mehrere der obengenannten Mischungspartner ausgefüllt wird.

Die zum Schutz der technischen Materialien verwendeten mikrobiziden Mittel oder Konzentrate enthalten den Wirkstoff bzw. die Wirkstoffkombination in einer Konzentration von 0,01 und 95 Gew.-%, insbesondere 0,lbis 60 Gew.-%.

Die Anwendungskonzentrationen der zu verwendenden Wirkstoffe bzw. der Wirkstoffkombinationen richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe bzw. Mittel ermöglichen in vorteilhafter Weise, die bisher verfügbaren mikrobiziden Mittel durch effektivere zu ersetzen. Sie zeigen eine gute Stabilität und haben in vorteilhafter Weise ein breites Wirkungsspektrum.

Die nachfolgenden Beispiele dienen zur Verdeutlichung der Erfindung. Die Erfindung ist nicht auf die Beispiele beschränkt.

### Herstellungsbeispiele

### Beispiel 1

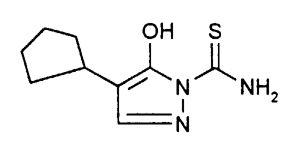

8 g 2-Formyl-2-cyclopentylessigsäureethylester, hergestellt aus der käuflichen 2-Cyclopentenessigsäure durch Verestern und Formylieren auf bekannten Wegen, wurden in 70 ml Ethanol mit 3,9 g Thiosemicarbazid 4 Stunden refluziert und nach Versetzen mit 4,8 g Kalium-tert.-butylat ca. 12 Stunden stehen gelassen. Aufgießen auf Eis / 10 % HCl ergibt 2,5°C der Zielverbindung vom Schmelzpunkt F: 160°C.

### Beispiel 2

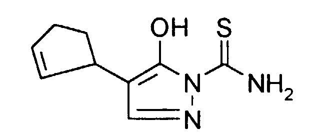

Analog Beispiel 1 erhält man die Verbindung vom Schmelzpunkt F. 152°C.

### Beispiel 2

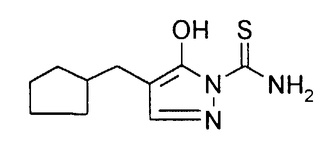

40,7 g 3-Cyclopentylpropionsäureethylester werden in 400 ml DMF bei 0 bis 5°C vorgelegt und mit 14,4 g Natriumhydrid (80 %ig in Öl) versetzt. Nach Zutropfen von 200 ml Ameisensäureethylester wird 24 h bei 25°C gerührt. Der Ansatz wird in 1,6 1 10 % HCl eingerührt, mit 800 ml Methylenchlorid extrahiert und destilliert. Man erhält 19,7 g (41 %) 2-Formyl-3-cydopentylpropionsäureethylester vom Siedepunkt 117°C/12 mm.

9,7 g des erhaltenen Öls wurden in 80 ml Ethanol mit 4,47 g Thiosemicarbazid 4 h unter Rückfluß erhitzt, bei 25°C mit 5,5 g Kalium-tert.-butylat versetzt und 24 h gerührt. Nach Eingeben in 250 ml 10 % HCI erhält man nach Umkristallisation aus Ethanol 5 g (45 %) der Zielverbindung vom Schmelzpunkt 157°C.

## Patentansprüche

1. Thiocarbamoyl-Verbindungen der Formel (I) 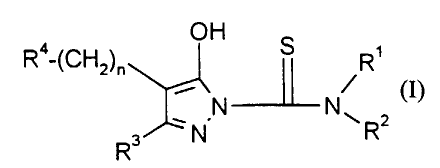 in denen
R¹, R², R³ jeweils unabhängig voneinander für Wasserstoff oder Methyl stehen und
R⁴ für Cyclopentyl oder Cyclopentenyl steht,
n für die Zahl 0 oder 1 steht,
sowie deren Metallsalzkomplexe und Säureadditionsprodukte.

2. Verbindungen der Formel (I) nach Anspruch 1, in denen R¹, R² und R³ für Wasserstoff stehen und n für die Zahl 0 steht.

3. Verbindung der Formel (I) nach Anspruch 1, in der R¹, R², R³ für Wasserstoff stehen, n für die Zahl 0 steht, R⁴ für Cyclopentyl steht.

4. Verfahren zum Schutz von technischen Materialien, dadurch gekennzeichnet, daß man Verbindungen der Formel (I) nach Anspruch 1 auf die zu schützenden Materalien aufbringt oder mit diesen vermischt.

5. Mittel zum Schutz von technischen Materialien enthaltend mindestens eine Verbindung der Formel (I) nach Anspruch 1.

6. Verwendung von Verbindungen der Formel (I) nach Anspruch 1 zum Schützen von Materalien gegen Befall und Zerstörung durch unerwünschte Mikroorganismen.

7. Verfahren zur Herstellung von Verbindungen der Formel (I), dadurch gekennzeichnet, daß man Formylsäurderivate der Formel (II) 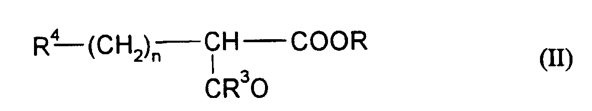 in denen n, R³ undR⁴ die in Anspruch 1 angegebenen Bedeutungen haben und R für Methyl, Ethyl, n-, i-Propyl oder n-, i-, s- oder t-Butyl steht mit Thiosemicarbaziden der Formel (III)
NH₂-NH-CS-NR¹R² (III)
gegebenenfalls in Gegenwart eines Verdünnungs- bzw. Lösungsmittels und/oder einer Base umsetzt.

## Claims

1. Thiocarbamoyl compounds of the formula (I) 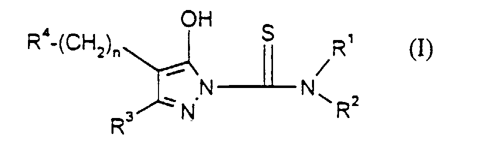 in which
R¹, R² and R³, in each case independently of one another, represent hydrogen or methyl, and
R⁴ represents cyclopentyl or cyclopentenyl,
n represents the number 0 or 1,
and their metal salt complexes and acid addition products.

2. Compounds of the formula (I) according to Claim 1, in which R¹, R² and R³ represent hydrogen and n represents the number 0.

3. Compound of the formula (I) according to Claim 1, in which R¹, R² and R³ represent hydrogen, n represents the number 0, R⁴ represents cyclopentyl.

4. Method of protecting industrial materials, characterized in that compounds of the formula (I) according to Claim 1 are applied to the materials to be protected or mixed with them.

5. Composition for protecting industrial materials, comprising at least one compound of the formula (I) according to Claim 1.

6. Use of compounds of the formula (I) according to Claim 1 for protecting materials against infestation and destruction by unwanted microorganisms.

7. Process for the preparation of compounds of the formula (I), characterized in that formyl acid derivatives of the formula (II) 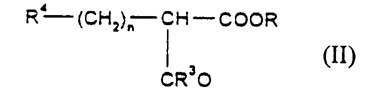 in which n, R³ and R⁴ have the meanings given in Claim 1 and R represents methyl, ethyl, n-, i-propyl or n-, i-, s- or t-butyl are reacted with thiosemicarbazides of the formula (III)
NH₂-NH-CS-NR¹R² (III)
if desired in the presence of a diluent or solvent and/or of a base.

## Revendications

1. Composés de thiocarbamoyle de la formule (I) 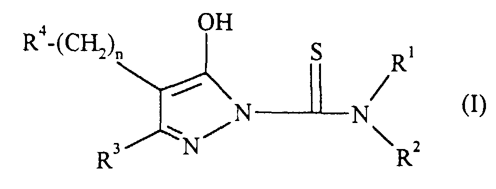 dans laquelle
R¹, R², R³ représentent à chaque fois indépendamment un atome d'hydrogène ou le groupe méthyle et
R⁴ représente le groupe cyclopentyle ou cyclopentényle,
n est le nombre 0 ou 1,
ainsi que leurs complexes de sels de métaux et produits d'addition avec des acides.

2. Composés de la formule (I) selon la revendication 1, dans lesquels R¹, R² et R³ représentent l'atome d'hydrogène et n est le nombre 0.

3. Composé de la formule (I) selon la revendication 1, dans lequel R¹, R², R³ représentent l'atome d'hydrogène, n est le nombre 0, R⁴ représente le groupe cyclopentyle.

4. Procédé pour la protection de matériaux techniques, caractérisé en ce que l'on applique des composés de la formule (I) selon la revendication 1 sur les matériaux à protéger ou on les mélange avec ceux-ci.

5. Agent de protection de matériaux techniques contenant au moins un composé de la formule (I) selon la revendication 1.

6. Utilisation de composés de la formule (I) selon la revendication 1 pour la protection de matériaux contre l'attaque et la destruction par des microorganismes non souhaités.

7. Procédé pour la préparation de composés de la formule (I), caractérisé en ce que l'on fait réagir des dérivés de l'acide formylique de la formule (II) 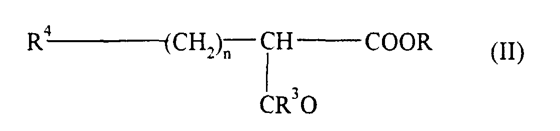 dans laquelle n, R³ et R⁴ ont les significations indiquées dans la revendication 1 et R représente le groupe méthyle, éthyle, n-, i-propyle ou n-, i-, s- ou t-butyle avec des thiosemicarbazides de la formule (III)
NH₂-NH-CS-NR¹R² (III)
éventuellement en présence d'un agent de dilution respectivement d'un solvant et/ou d'une base.
